(19) Europäisches Patentamt
European Patent Office
Office européen des brevets

(11) EP 0 791 849 A1

(12) **EUROPEAN PATENT APPLICATION**

(43) Date of publication:
**27.08.1997 Bulletin 1997/35**

(51) Int. Cl.[6]: **G02F 1/35**

(21) Application number: **97100845.3**

(22) Date of filing: **21.01.1997**

(84) Designated Contracting States:
**AT BE CH DE DK ES FR GB GR IE IT LI LU NL PT SE**

(30) Priority: **26.02.1996 US 606972**

(71) Applicant: **ENICHEM S.p.A.**
**20124 Milano (IT)**

(72) Inventors:
- **Jen, Kwan-Yue Alex**
  **Old Bridge - N.Y. 08857 (US)**
- **Drost, Kevin Joel**
  **Lafayette, Indiana 47905 (US)**

(74) Representative: **Gennari, Marco**
**Eniricerche S.p.A.,**
**BREL,**
**Via F. Maritano, 26**
**20097 San Donato Milanese (MI) (IT)**

# (54) Non-linear optical compounds

(57) Nonlinear optical compounds exhibiting second order nonlinear optical properties and possessing delocalized resonance configurations corresponding to:

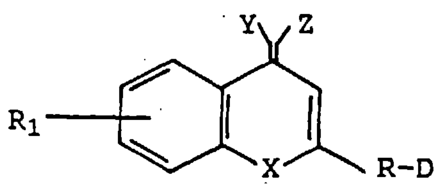

wherein R is a pi-conjugated electron delocalized organic moiety; D is an electron donating group; $R_1$ is selected from hydrogen, electron withdrawing moieties, polymer attachment groups, -CN, -COOR'', -SOR'' and -$SO_2R''$, wherein R'' is an alkyl group containing from 1 to 15 carbon atoms; X is O or S; and Y and Z are independently selected from CN, $CO_2R'$, $NO_2$, COR' and $SO_2R'$, wherein R' is an alkyl group containing from 1 to 15 carbon atoms, or X and Y together form a ring structure selected from 1,3-indanediones, N,N-diallyl and N,N-dialkylthiobarbituric acids, 3-dicyanovinylindane-1-ones, 1,3-bisdicyanovinyl indanes and 3-sulfone-1-dicyanovinyl indanes. Polymers blended with, cured with, or having pendant side chains of the disclosed nonlinear optical compounds and exhibiting second-order nonlinear optical properties are also disclosed.

EP 0 791 849 A1

## Description

The present invention relates to aromatic and heteroaromatic compounds with nonlinear optical (NLO) properties. In particular, the present invention relates to NLO compounds having improved chemical and thermal stability.

The compounds of the present invention are stable in processing solvents and at processing temperatures used in the production of electro-optic devices. When suitably oriented, the compounds are capable of highly efficient second harmonic generation and electro-optic modulation of an electromagnetic wave having a wavelength between 300 nm and 2,000 nm. The present invention further relates to polymer compositions of the disclosed compounds.

Highly efficient NLO materials capable of doubling or tripling the frequency of incident light are currently of great scientific and technological interest for use in optical telecommunications, signal processing and the construction of optical computers. Nonlinear optics is concerned with the interaction of electromagnetic fields in various media to produce new fields which may be altered in phase, frequency or amplitude. The NLO effect of a material upon an electromagnetic field is a function of the second and higher order terms of the following equation:

$$P=\alpha E+\beta E^{2}+\gamma E^{3}+...$$

P is the polarization of a material, E is the intensity of electric field, and the coefficients $\alpha$, $\beta$ and $\gamma$, etc. are indicative of the NLO susceptibility of the material. Such coefficients are constant for a given material, but vary from material to material. The second order coefficient, $\beta$, for a given material, is indicative of the second harmonic generation properties of the material, with second harmonic generation efficiencies increasing as the value of $\beta$ increases.

Candidate NLO materials should possess good physical properties, such as high optical transparency, low dielectric constant and high laser damage threshold. The materials should also possess the molecular nonlinearity required of NLO materials, in particular, high $\beta$ values, fast response times and nonlinear susceptibility over a broad range of wavelengths, particularly of wavelengths between about 300 nm and 2,000 nm.

Recent efforts in the development of NLO materials have focused upon non-centrosymmetric organic materials with large delocalized pi-electron systems, which exhibit great nonlinear susceptibilities and can be varied to optimize the desired physical and mechanical properties. This includes the single benzene ring derivative disclosed by U.S. Patent No. 4,894,186 to Gordon and the compounds derived from two to four benzene rings separated by pi-electron conjugated carbon-carbon, carbon-nitrogen and nitrogen-nitrogen bridges disclosed by U.S. Patent No. 4,892,681 to Myata et al., U.S. Patent No. 4,894,263 to Dubois et al., U.S. Patent No. 4,933,112 to DeMartino et al. and U.S. Patent No. 4,935,292 to Marks et al.

To induce charge asymmetry, and consequently second order nonlinear polarizability, an aromatic ring at one end of the NLO compound structure is substituted with an electron donating group, while on the other end of the NLO compound structure an aromatic ring is substituted with an electron accepting group. The dipole of the compound structure can then be aligned in accordance with the method described by U.S. Patent No. 4,935,292, the disclosure of which is hereby incorporated herein by reference thereto.

However, pi-electron conjugated bridges linking the aromatic or heteroaromatic rings of NLO compounds are a source of thermal and photochemical instability. The stability of non-centrosymmetric organic materials with large delocalized pi-electron systems in processing solvents and host polymers at processing temperatures is an important parameter in their application in electro-optic devices. Because high-$T_g$ polyimide based electro-optic polymers are likely candidates to be used in the production of NLO devices, candidate NLO compounds must survive the stringent processing conditions required to produce stable electro-optic polyimides. The NLO compounds must be able to withstand temperatures in excess of 300°C. The compounds must also survive being in a polyamic acid while it is being cured to a polyimide. In most cases, this transition entraps the reaction solvent, typically N-methylpyrrolidone (NMP), dimethylacetamide (DMAC), dimethylformamide (DMF), and the like, together with superheated water vapors that, in combination with the polar solvent, create very acidic conditions.

NLO compounds that are stable in solvents such as NMP, DMAC and DMF, both at room temperature and at boiling temperatures, are not well established in the literature. High temperature stability is not enough. Molecules that are inherently stable at 300°C are not necessarily stable in polyamic acids while being cured to polyimides. Many compounds decompose near the $T_g$ of the polymers. The general instability of NLO compounds in the solvents is attributable to the sensitivity of the electron accepting groups toward the solvent or solvent decomposition products, typically open chain amine-type basic impurities induced by either light, oxygen or heat.

Near the polymer $T_g$, the solvent or solvent decomposition products that have become trapped in the polymer matrix diffuse out of the matrix and cause decomposition of the NLO compounds. The NLO compounds that decompose consist of a variety of systems containing dicyanovinyl, nitro and tricyanovinyl electron accepting groups. Accordingly, a need exists for NLO compounds with less-reactive electron accepting groups that are stable under polyimide curing and processing conditions without sacrificing NLO activity.

This need is met by the present invention. NLO compounds have now been discovered containing substituted chromone electron accepting groups that are chemically and thermally stable under polyimide processing conditions.

Therefore, in accordance with the present invention, there is provided a nonlinear optical compound exhibiting second-order nonlinear optical properties and possessing a delocalized resonance configuration having a structure represented by Formula I:

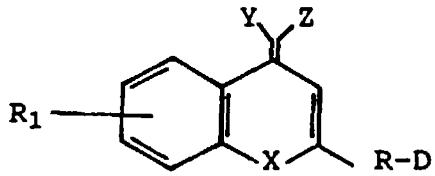

(I)

in which R is a pi-conjugated electron delocalized organic moiety; D is an electron donating group; $R_1$ is selected from hydrogen, electron withdrawing moieties, polymer attachment groups, -CN, -COOR", -SOR" and $-SO_2R"$, wherein R" is an alkyl group containing from 1 to 15 carbon atoms; X is S or O; and Y and Z are independently selected from CN, $CO_2R'$, $NO_2$, COR' and $SO_2R'$, wherein R' is an alkyl group containing from 1 to 15 carbon atoms, or Y and Z together form a ring system selected from 1,3-indane diones, N,N-diallyl and N,N-dialkyl thiobarbituric acids, 3-dicyanovinyl indane-1-ones, 1,3-bisdicyano-vinyl indanes and 3-sulfone-1-dicyanovinyl indanes.

R can be any pi-conjugated electron delocalized organic moiety exhibiting optical nonlinearity. R may contain from 1 to 10 aromatic rings or fused ring systems, linked together so as to form a delocalized resonance configuration. Suitable linkages include pi-electron conjugated carbon-carbon, carbon-nitrogen and nitrogen-nitrogen bridges. At least one five- or six-membered heteroaromatic ring is preferably present, alone, or as part of a fused ring system, which heteroaromatic ring contains at least one heteroatom selected from O, N, S or Se. The number or size of the fused ring systems should not be so large as to interfere with the solubility of the NLO compounds in polymer matrices or processing solvents.

The NLO compounds of the present invention are uniquely stable under the curing conditions of polyamic acids in solvents such as NMP, and are stable in polyimides at temperatures above 300°C, attributable to the use of a chromone electron accepting group in place of a nitro or di- or tricyanovinyl electron accepting group. Compounds with chromone electron accepting groups have lowered $\beta\mu$ values than similar compounds containing a tricyanovinyl accepting group, because of the weaker electron withdrawing properties of chromone-based electron accepting groups. However, the $\beta\mu$ of the compounds can be varied greatly by adjusting the X and Y groups of the chromone system. A series of known electron withdrawing groups have been incorporated into the chromone system, and by employing chromone electron accepting groups with heteroaromatic ring conjugating units and efficient electron donating groups, improved solubility as well as chemical and thermal stability can be achieved without a significant sacrifice of molecular nonlinearity.

Another embodiment of the present invention provides a combination exhibiting second order NLO properties. This combination includes the NLO compounds of the present invention and a material chemically inert thereto. The NLO compounds of these combinations preferably have an external field-induced molecular alignment.

In one aspect of this embodiment of the invention, the NLO compound is disposed as a layer or layers on a substrate of a material chemically inert thereto, such as glass, silica, silicon and polymeric materials. In another aspect of this embodiment of the invention, the NLO compound is in the form of a blend of a guest compound in a host matrix, with the NLO compound of the present invention serving as the guest compound and the material chemically inert thereto serving as the host matrix. The material that is chemically inert to the NLO compound is preferably a thermoplastic polymer selected from polyacrylates, polymethacrylates, polyurethanes, polyquinolines, epoxy polymers, polybenzoxazoles, polybenzothlazoles, polysulfones, polyacrylamides, polycarbonates, polyamides, polyesters, polystyrenes, polyimides, polyether ketones, polyether ether ketones, polyphenylene ethers and copolymers thereof.

In still yet another aspect of this embodiment of the present invention, pendant side chains of the NLO compounds of the present invention are covalently bonded to a polymer material chemically inert thereto. In accordance with a preferred aspect of this embodiment of the invention, $R_1$ of the compound of Formula I is a polymer attachment group, preferably a functionalized alkyl moiety, that is covalently linked to a polymer. The polymer contains one or more monomeric subunits having a reactive group capable of being covalently attached to the polymer attachment group, whereby the compound of Formula I is covalently linked to the polymer at the reactive group of one of the subunits via the polymer attachment group. A polymer attachment group may be located on the donor side of the NLO compound as well, with or without $R_1$ being a polymer attachment group.

Preferably, the polymer contains a plurality of the monomeric subunits having the reactive groups covalently substituted with an NLO compound via a polymer attachment group, so that the ratio of the monomeric subunits having reactive groups covalently linked to an NLO compound to monomeric subunits without an NLO compound covalently linked thereto is between 1:99 and about 50:50. The monomeric subunits having a reactive group capable of being covalently attached to a polymer attachment group and the monomeric subunits without an NLO compound covalently linked thereto are independently selected from polyacrylate, polyimide, polyquinoline, polybenzoxazole, polyurethane,

polybenzothiazole, polysulfone, polyamide, polyacrylamide, polystyrene, polyvinyl halide, polyacrylonitrile, polyvinyl alcohol, polyvinyl acetate, polyester, polyethylene, polypropylene, polyisobutylene, polyisoprene, poly(acid anhydride) and polycarbonate monomeric subunits.

In yet another aspect of this embodiment of the present invention, the NLO guest compounds of the present invention are cross-linked within the host matrix. The NLO compound may function as the cross-linking agent that forms the host matrix, or the NLO compound may simply function as a guest compound within a cross-linked host matrix.

For the NLO compound of Formula I to function as a cross-linking agent, $R_1$ may be a polymer attachment group, or either or both of R and D are also substituted with a polymer attachment group. Polymers capable of being cross-linked by an NLO compound in accordance with this aspect of the present invention include polyacrylates, polymethacrylates, polyurethanes, polyquinolines, epoxy polymers, polybenzoxazoles, polybenzothiazoles, polysulfones, polyacrylamides, polycarbonates, polyamides, polyesters, polystyrenes, polyimides, polyether ketones, polyether ether ketones, polyphenylene ethers and copolymers thereof.

Polymers that are capable of being independently cross-linked to form a host matrix for an NLO guest compound include epoxy polymers, polyimides, polyurethanes, polyamides, polyacrylates, and the like.

The use of chromone electron accepting groups with pi-conjugated electron delocalized organic moieties provides an efficient method to enhance molecular nonlinearity while increasing the thermal and chemical stability of the NLO compounds as well. Furthermore, the use of heteroaromatic conjugating units with chromone electron accepting groups allows for a large variation of molecules possessing heretofore unattained chemical and thermal stability, without sacrificing second order nonlinearity. In addition to possessing good second order molecular nonlinearity and thermal and chemical stability, the compounds of the present invention are soluble in polymer matrices and spin-casting solvents, have high laser damage thresholds, are easily synthesized and have well-known and understood chemical properties.

The NLO compounds of the present invention, once suitably oriented, exhibit a high second order NLO susceptibility. Compounds suitable for use as second order NLO chromophore materials according to the present invention have the structure of Formula I in which R can be any pi-conjugated electron delocalized organic moiety.

Suitable organic moieties contain from 1 to 10 aromatic rings or fused ring systems. Two or more rings or fused ring systems are linked together to form a delocalized resonance configuration.

Within the present specification "aromatic" rings are defined as aromatic carbocyclic rings such as benzene rings. "Heteroaromatic" rings refer to aromatic heterocyclic rings, thereby excluding aromatic carbocyclic rings such as benzene and nonaromatic heterocyclic rings such as pyrrolidine.

The electron delocalized organic moieties of the present invention preferably contain from one to four aromatic rings or fused ring systems. The aromatic rings or fused ring systems within each electron delocalized organic moiety may be the same or different.

For organic moieties containing multiple rings or fused ring systems, it is preferably that at least one ring alone, or within a fused ring system, be a five-membered heteroaromatic ring having one heteroatom selected from O, N, S, Se and Te. The heteroaromatic ring may optionally include up to three additional N atoms. Preferably, the five-membered heteroaromatic rings possess a structure corresponding to Formula II:

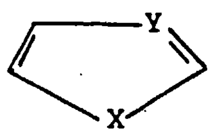

(II)

in which Y is C or N and X is selected from O, N, N-alkyl, S, Se and Te.

Preferably, the organic moieties containing multiple rings or fused ring systems contain two or more of the five-membered heteroaromatic rings, alone or as part of a fused ring system. Most preferably, all the rings in organic moieties containing multiple rings or fused ring systems are five-membered heteroaromatic rings, and all fused ring system contain a five-membered heteroaromatic ring. When two or more heteroaromatic rings are present in an electron delocalized organic moiety, the rings may have the same or different heteroatoms.

The fused ring systems, when present, should not be so large as to hinder the solubility of the NLO compounds in polymer matrices or spin-casting solvents. Likewise, the value of n should not be so high as to interfere with solubility. The point at which the number of single or fused rings, or fused ring system size, interferes with solubility is easily identified by those of ordinary skill in the art.

For organic moieties containing multiple rings or fused ring systems, adjacent rings or fused ring systems are preferably linked by from one to three pi-electron conjugated functional groups such as carbon-carbon, carbon-nitrogen or nitrogen-nitrogen functional groups. Preferably, the adjacent rings or fused ring systems are bridged by one or two of the conjugated functional groups. When adjacent rings or fused ring systems are bridged by two or three functional groups; the conjugated functional groups may be the same or different and the number of conjugated functional groups between adjacent rings or fused ring systems may vary within an NLO compound. When the ring is heteroaromatic or

the fused system contains a heteroaromatic ring, the linkage is preferably substituted on the ring alpha to a heteroatom. For six-membered rings, alone, or within fused ring systems, the linkage is substituted para to another linkage, an electron donating group or the chromone electron accepting group.

The use of pi-electron conjugated functional groups to bridge adjacent rings or fused ring systems in NLO compounds is essentially conventional to the art of the NLO active organic materials. Examples of suitable ring- or fused ring system-bridging functional groups known in the art include -N=N, -CH=N-, -CH=N-N=CH-, -C≡C- and $(CH=CH-)_j$, with j being from one to three.

The adjacent rings or fused ring systems of the electron delocalized organic moieties having linked aromatic rings or fused ring systems may also be linked by non-conjugated linkages. The adjacent rings or fused ring systems may also be covalently bonded between single ring members to directly link the rings or fused ring systems without forming a fused structure of the adjacent rings or fused ring systems.

The pi-conjugated electron delocalized organic moieties of the present invention represented by R in Formula I can also include a single aromatic ring or fused ring system. The preferred single ring is a five-membered heteroaromatic ring as defined above. Fused rings are preferred over single rings, and fused ring systems containing at least one five-membered heteroaromatic ring, as defined above, are most preferred. The fused ring systems of the present invention are limited to two- and three-ring fused ring structures so as not to hinder the solubility of the NLO compounds in polymer matrices or processing solvents.

The configuration of multiple heteroaromatic rings within a fused ring system is not critical, and may be an all "up" configuration or an alternating "up" and "down" configuration, as depicted in the above-cited U.S. Patent Application Serial Number 930,732, the disclosure of which is hereby incorporated herein by reference thereto.

The fused ring systems of the present invention are not limited to structures containing only five-membered heteroaromatic rings. Two-ring fused ring systems suitable for use with the present invention may also contain a benzene or pyridine ring. Three-ring fused ring systems suitable for use with the present invention may Contain up to two benzene or pyridine rings, or a benzene and a pyridine ring, in addition to the five-membered heteroaromatic ring. Thus, the fused ring systems of the present invention include such structures depicted in the above-mentioned U.S. Patent Application Serial Number 930,732. When a two- or three-ring system includes pyridine, the pyridine should not be quaternized. Such ionic species cause severe current leakage during the dipole-alignment electric field poling process.

From the foregoing description, the aromatic and fused ring systems suitable for use with the present invention can be easily identified by those of ordinary skill in the art. Suitable rings and ring systems include, but are not limited to, pyrrole, furan, thiophene, imidazole, oxazole, thiazole, triazole, tetrazole, pyrazole, pyrimidine, purine, quinolines, carbazole, benzene, naphthalene, furazan, pyrazine, indole, isoindole, indazole, phenothiazine, benzotriazole, anthracene, phenanthrene, azophenanthrenes, quinazolines, pteridine, and the like.

To induce charge asymmetry, the NLO compounds of the present invention contain at least one electron donating group and a chromone electron accepting group containing two electron withdrawing moieties. The portion of the compound of Formula I that functions as an electron accepting group is depicted below as Formula IA:

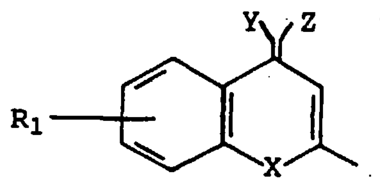

(IA)

Y and Z represent electron withdrawing moieties. The compound of Formula I also contains at least one electron donating group, represented by D.

The electron accepting chromone and electron donating group or groups should be ring-substituted on R so as to form a delocalized resonance configuration. Positions for substituting electron donating groups to form delocalized resonance configurations can be readily determined by those of ordinary skill in the art.

When R represents a fused ring system containing five-membered heterocyclic rings, the electron donating and electron accepting groups are preferably substituted on the five-membered heterocyclic ring members of the fused ring systems, although this is not essential. When substituted on five-membered heterocyclic rings, the electron donating group or electron accepting group is preferably substituted alpha to a heteroatom. Regardless of whether the fused ring system contains a five-membered heteroaromatic ring, the electron donating and accepting groups are preferably substituted to ring members of different rings so as to maximize electron delocalization. Examples of typical delocalized resonance configurations are depicted in the above-cited U.S. Patent Application Serial Number 930,732.

In the chromone electron accepting group depicted in FIG. IA, X is O or S and Y and Z are independently selected from $-NO_2$, -CN, -COR', -COOR' and $-SO_2R'$, wherein R' is an alkyl group containing from 1 to 15 carbon atoms. Y and Z may also together form a ring structure. Preferred ring structures include N,N-diallyl and N,N-dialkyl thiobarbituric acids, indane-1,3-diones, 3-dicyanovinyl indane-1-ones, 3-sulfone-1-dicyanovinyl indanes and 1,3-bisdicyano-vinyl

indanes. X and Y are preferably either both -CN or together form the above-listed ring structures.

The benzene ring of the chromone electron accepting group may optionally be further substituted. That is, $R_1$, instead of being hydrogen, may be an electron withdrawing moiety, a polymer attachment group, or, for example, a substituent selected from CN, COOR", SOR", $SO_2R$", wherein R" is an alkyl group containing from 1 to 15 carbon atoms. $R_1$ should be ring-substituted so as to maintain the delocalized resonance configuration of the NLO compound.

Electron donating groups that are used to induce charge asymmetry to the structure of Formula I are essentially conventional to the art of NLO active organic materials. Guidance for the selection of electron donating groups can also be found in Nicoud et al., Ch. II-3 of <u>Nonlinear Optical Properties of Organic Molecules and Crystals</u>, Vol. 1, (Chemla and Zyss, Eds., Academic Press, Inc., New York 1987), p.233. Essentially any functional group capable of releasing electrons into the pi-electron system of an aromatic or heteroaromatic ring or fused ring structure is suitable for use as the electron donating group D.

Examples of suitable electron donating groups known in the art include $-NR_6R_7$, $-OR_8$, $-SR_8$, $-TeR_8$, $-SeR_8$, $-CH=NR_9$, $-CH=N-NR_6R_7$, and $-CH=C[N(R_6R_7)]_2$, wherein $R_6$ and $R_7$ are independently selected from hydrogen, alkyl groups containing up to 12 carbon atoms and alkyl groups containing up to 12 carbon atoms having reactive functional groups selected from hydroxyl, ethylene, acetylene, amine, thiol, sulfonic acid, carboxylic acid, or $R_6$ and $R_7$ together form a cyclic group containing up to 8 carbon atoms, including groups such as pyrrolidine, piperidine, piperazine and morpholine. $R_8$ is an alkyl group containing up to six carbon atoms and $R_9$ is hydrogen or an alkyl group containing up to 10 carbon atoms. Preferably, $R_6$ and $R_7$ are independently selected from methyl, ethyl, hexyl, cyclopentyl, cyclohexyl, pyrrolidine, piperadine, piperazine and morpholine, $R_8$ is preferably a methyl group and $R_9$ is preferably either hydrogen or a methyl group.

Another example of suitable electron donating groups are the functional groups:

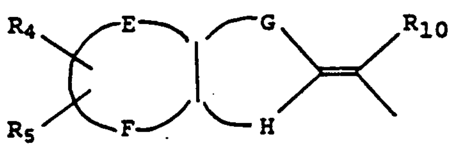

and

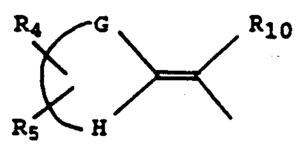

wherein E, F, G and H are members of a saturated or unsaturated five- to eight-membered cyclic ring or two-ring system having five- to eight-membered rings that are electron donating in nature. E, F, G and H are heteroatoms independently selected from -CH-, $-CH_2-$, O, N, S, Se, Te and $-NR_3-$. $R_3$, $R_4$, $R_5$ and $R_{10}$ are independently selected from hydrogen, alkyl moieties containing up to 18 carbon atoms and functionalized alkyl moieties containing up to 18 carbon atoms, wherein the functionalized alkyl moieties are selected from alkoxy, aminoalkyl, alkylhalide, hydroxyalkyl, alkylsulfide, alkylisocyanate, alkylisothiocyanate, alkylthiol, alkylazide, alkylcarboxylic, alkylsulfonic, alkylalkene and alkylalkyne moieties.

Examples of suitable one- or two-ring electron donating groups include dithiane and dithiolium groups such as 1,3-dithiolium, 2-benzo-1,3-dithiolium and 2-ethylenedithio-1,3-dithiolium, and the like. Whether or not a ring is electron donating in nature to meet the definition of membership in the group is understood by those of ordinary skill in the art.

Strong electron donating groups are preferred, which significantly increase the second order NLO properties of the compounds of the invention. Examples of strong electron donating groups are $-N(CH_3)_2$, pyrrolidine, dithiane, piperidine, piperazine, morpholine and dithioliums such as 1,3-dithiolium, 2-benzo-1,3-dithiolium and 2-ethylenedithio-1,3-dithiolium. The most preferred strong electron donating group is 2-ethylenedithio-1,3-dithiolium.

The aromatic or heteroaromatic rings or fused ring systems of the NLO compounds of the present invention may optionally be further substituted. Any number of functional groups can be substituted on the aromatic or heteroaromatic ring or rings, provided that the groups are not so large or so numerous to cause undesirable steric hindrance effects, the occurrence of which will be clear to those of ordinary skill in the art.

The preferred embodiment of the present invention includes a second electron donating group attached to the same ring or ring member of a fused ring system as the first electron donating group described above, so that both electron donating groups, the chromone electron accepting group and the electron delocalized organic moiety R form a delocalized resonance configuration. The second electron donating group may be the same or different than the first electron donating group. The inclusion of a second electron donating group increases the second order NLO properties of the resulting compound as compared to compounds having single-substitution of electron donating groups.

The NLO compounds of the present invention may optionally include up to two polymer attachment groups. These groups are functionalized substituents that serve as means for attaching the NLO compound to a polymer chain. The presence of one polymer attachment group allows the covalent attachment of the NLO compounds of the present invention as side chains to the monomeric subunits with polymers. When two polymer attachment groups are present, the NLO compounds are capable of cross-linking matrix polymers.

Thus, $R_1$ can be a functionalized moiety capable of covalently linking the NLO compound to a monomeric subunit of a polymer by conventional addition or condensation reactions. Alternatively, or at the same time, D can be an electron donating group functionalized with an appropriate substituent for covalent attachment of the NLO compound to a polymer subunit. The aromatic or heteroaromatic rings or fused ring systems of the present invention can also be functionalized with appropriate substituents for covalent attachment of NLO compounds to polymer subunits. Substituent groups suitable for the covalent attachment of NLO compounds to polymer subunits are well-known to those of ordinary skill in the art. Examples of polymer attachment groups include functionalized alkyl moieties containing up to 18 carbon atoms, wherein the functionalized alkyl moieties are selected from alkoxy, aminoalkyl, alkylhalide, hydroxyalkyl, alkylsulfide, alkylisocyanate, alkylisothiocyanate, alkylthiol, alkylazide, alkylcarboxylic, alkylsulfonic, alkylalkene and alkylalkyne moieties.

Preferred polymer attachment groups will also increase the solubility of the materials both in polymer matrices and in spin-casting solvents. Ordinarily, the solubility would decrease as the number of rings or fused ring systems in the non-centrosymmetric organic moiety increases. Substituents that will increase the solubility of the non-centrosymmetric organic moieties are well-known to those of ordinary skill in the art. Preferred substituents include alkyl moieties containing between 3 and 12 carbon atoms and functionalized alkyl moieties containing between 3 and 12 carbon atoms, wherein the functionalized alkyl moieties are selected from alkoxy, aminoalkyl, alkylhalide, hydroxyalkyl, alkylsulfide, alkylthiol, alkylazide, alkylcarboxylic, alkylsulfonic, alkylalkene, alkylisocyanate, alkyl-isothiocyanate and alkylalkyne moieties.

The aromatic ring systems upon which the NLO compounds of the present invention may be based are prepared by well-known methods widely reported in the prior art. The preparation of many of these moieties is disclosed in the above-cited U.S. Patent Application Serial Numbers 626,358 and 930,732. Some of the rings and fused ring systems are commercially available. The chromone electron accepting group containing the electron withdrawing moieties Y and Z and the electron donating group can be substituted to the aromatic or heteroaromatic rings or fused ring systems using conventional methods.

A general procedure for the preparation of the NLO compound of the present invention, in which an electron donating group substituted aromatic or heteroaromatic aldehyde is reacted with a 2'-hydroxy-2-(methyl-sulfinyl) acetophenone as illustrated by the following reaction scheme:

Scheme I

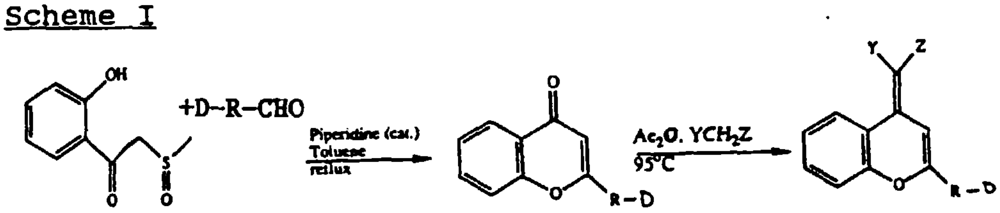


R, $R_1$, D, X, Y and Z are the same as described above with respect to Formula I. The preparation of 2'-hydroxy-2-(methyl-sulfinyl) acetophenone from ethyl salicylate is shown in Example I.

The NLO compounds of the present invention can be formed into a nonlinear optical material by combining the NLO compounds with a medium that is chemically inert to the compounds. For example, the NLO compounds can be layered on a substrate such as glass, silica or polymeric materials, as described in U.S. Patent No. 4,894,186 to Gordon, the disclosure of which is hereby incorporated herein by reference thereto.

In another embodiment, a nonlinear optical medium can be formed by blending the NLO compounds of the present invention with a host thermoplastic polymer. Suitable host thermoplastic polymers include polyacrylates, polymethacrylates, polyurethanes, polyquinolines, epoxy polymers, polybenzoxazoles, polybenzothiazoles, polysulfones, polyacrylamides, polycarbonates, polyamides, polyesters, polystyrenes, polyimides, polyether ketones, polyether ether ketones, polyphenylene ethers and copolymers thereof. This combination is also described in U.S. Patent No. 4,894,186, the disclosure of which is also hereby incorporated herein by reference thereto.

The NLO compounds of the present invention can also be covalently attached as side chains to the monomeric subunits of polymers. Polymers should be chosen for use with the present invention having monomeric subunits that have reactive functional groups for attachment of side chains. The polymer should have excellent optical transparency, good film-forming characteristics, a low dielectric constant and a relatively high $T_g$ for stable dipole orientation of the side chains. Other properties will come into consideration, depending upon the particular end-use requirements of the material; however, these properties are well-understood by those of ordinary skill in the art.

One class of polymers suitable for use with the present invention are polymers and copolymers, the monomeric subunits of which are independently selected from polyacrylate, polymethacrylate, polyimide, polyquinoline, polybenzoxazole, polyurethane, polybenzothiazole, polysulfone, polyamide, polyacrylamide, polystyrene, polyvinyl halide, polyacrylonitrile, polyvinyl alcohol, polyvinyl acetate, polyester, polyethylene, polypropylene, polyisobutylene, polyisoprene, poly(acid anhydride) and polycarbonate monomeric subunits.

The polyacrylates suitable for use with the present invention include alkyl branched polyacrylates such as polymethacrylates. Likewise, the polyacrylamides suitable for use with the present invention include alkyl branched polyacrylamides such as polymethacrylamide, and the polyacrylonitriles include alkyl branched polyacrylonitriles such

as polymethacrylonitrile.

Those of ordinary skill in the art are capable of identifying the functional groups of the polyacrylates, polyamides, polyacrylamides, polyvinyl-halides, polyacrylonitriles, polyvinyl alcohols, polyvinyl acetates, polyesters, polyphenylene ethers, polyether imides, polyether ketones, polyether ether ketones, polyacid anhydrides and polycarbonates to which the NLO compound of the present invention can be attached by conventional addition and condensation reactions.

Although the monomeric subunits of polyimides, polystyrene, polyethylene, polypropylene, polyisobutylene and polyisoprene do not have such functional groups, such monomeric subunits can first be functionalized to form a reactive group for the attachment of the NLO compound. See, for example, the chloromethylation of polystyrene and the subsequent conversion to the more reactive iodomethyl derivative set forth in U.S. Patent No. 4,935,292 to Marks, the disclosure of which is herein incorporated by reference thereto.

Alternatively, a functionalized derivative of these polymers can be used as a starting material, such as the poly(p-hydroxystyrene), the use of which is also disclosed by U.S. Patent No. 4,935,292. As another alternative, rather than covalently bond the materials, the polymers can be polymerized in the presence of the NLO compounds of the present invention so that a host polymer matrix is formed within which the NLO compound is present as a guest molecule.

The NLO compounds of the present invention are attached to the monomeric subunits by the polymer attachment groups discussed above. The polymer attachment group is covalently linked to the polymer at a reactive functional group of one of the monomeric subunits suitable for attachment of the NLO compound. Thus, the NLO compounds are covalently linked to a polymer at the reactive groups of the monomeric subunits via polymer attachment groups on the NLO compounds.

The NLO compounds of the present invention can be attached as polymer side chains by using a single polymer attachment group to covalently bond the NLO compounds to monomeric subunits of the polymer. The NLO compounds can also be attached between two or more monomeric subunits using multiple polymer attachment groups, thereby cross-linking polymer chains.

Polymers in accordance with this aspect of the present invention will not be completely substituted with NLO groups. The present invention includes polymers having ratios of NLO substituted monomeric subunits to unsubstituted monomeric subunits between about 1:99 and about 50:50. Substitution ratios between about 5:95 and about 40:60 are preferred. Substitution ratios less than about 30:70 are more preferred in order that the polymer remains soluble in solvents utilized in the preparation of NLO materials. The most preferred substitution ratio is about 25:75.

In preferred NLO polymers, the monomeric subunits are independently selected from monomers of ethylene, acrylates, alkyl-branched acrylates, poly-imides, acrylamides, alkyl-branched acrylamides, styrenes, alpha-alkyl styrenes, vinyl acetate, ether ketones and ether ether ketones. When one of the monomeric subunits is a styrene monomer, the aromatic ring may be further substituted by one or more hydroxyl or alkyl groups, provided that the groups are not so large or so numerous to cause undesirable steric hindrance effects, the occurrence of which will be clear to those or ordinary skill in the art. In the most preferred NLO polymers, the monomeric subunits are independently selected from monomers of acrylates, methacrylates, polyimides, ether ketones and ether ether ketones.

The polymerization of the polymeric NLO compounds of the present invention is essentially conventional and readily understood by those of ordinary skill in the art. Depending upon the material in question, in some cases, it is preferable first to polymerize the polymer and then attach the side chains to the reactive groups of the monomeric subunits. In other cases, it is preferable to synthesize a monomer having an NLO side chain covalently attached thereto to be copolymerized with a monomer having no side chain. The NLO polymers are then recovered and purified by conventional means known to those of ordinary skill in the art.

The NLO compounds of the present invention are particularly well-suited for use with polyimides. Polyimides are formed by the reaction of an aromatic diamine with an aromatic dianhydride to form a polyamic acid, which then undergoes a ring-closing reaction to form the polyimide structure. The cyclization to the polyimide is accomplished by a thermally induced intramolecular condensation. This is performed at elevated temperatures above 300°C in solvents like NMP, DMAC and DMF, for which the NLO compounds of the present invention are particularly well suited because of their thermal and chemical stability under these conditions.

The NLO compounds of the present invention may simply be dissolved in polyamic acid solutions, which are then cured to form a polyimide matrix containing the NLO compounds. The polyimide may be thermoplastic or it may be thermosetting. The NLO compounds of the present invention may also be covalently attached to the polyimide by formation of a polyamic acid with NLO side chain-substituted aromatic rings that is subsequently cured to form the polyimide. Polyamic acids having NLO side chain substituted aromatic rings are obtained by forming the polyamic acid from aromatic diamines or aromatic dianhydrides, or both, that are ring-substituted with side chains of the NLO compounds of the present invention. This is described in co-pending and commonly owned U.S. Patent Application Serial No. 08/500,384, the disclosure of which is incorporated herein by reference.

The NLO compounds of the present invention can be linked to two aromatic diamines or two aromatic dianhydrides, or to an aromatic diamine and an aromatic dianhydride so as to form a cross-linked polyamic acid. The cross-linked polyamic acid is then cured to form a polyimide cross-linked by the NLO compounds of the present invention.

The preferred NLO polymers of the present invention typically have weight-average molecular weights between

about 5,000 and about 300,000 daltons measured by GPC or light scattering. Films of the polyimides and other polymers may be formed by spin-coating, after which the films may be repetitively annealed prior to poling at an elevated temperature at the $T_g$ of the material. Following annealing, the dipoles of the side chains may be aligned by application of an intense electric field, (0.2-3.0 MV $cm^{-1}$) at temperatures near the $T_g$.

Because of the high $T_g$'s of polyimides, the annealing and dipole aligning steps for these polymers is typically performed at temperatures greater than 250°C. The NLO compounds of the present invention possess the chemical and thermal stability required for these conditions. Such conditions are also encountered when forming electro-optic devices from polyimide NLO compounds of the present invention. The foregoing sequence of spin-coating, annealing and poling is essentially conventional and disclosed in U.S. Patent No. 4,935,292, the disclosure of which is hereby incorporated herein by reference thereto.

It is disclosed in U.S. Patent No. 4,935,292 and SPIE Proceeding No. 1147, 74-83 (1989) that for non-cross-linked polymers, further stabilization of the NLO side chain alignment can be achieved by radiation-induced or chemical-induced cross-linking of the polymer matrix. This process is also essentially conventional, and the disclosure of which in U.S. Patent No. 4,935,292 is also hereby incorporated herein by reference thereto.

The electro-optic coefficient of an NLO-active poled polymer film is proportional to the product of the molecular second order nonlinear optical susceptibility coefficient, β, and the molecular ground state electric dipole moment, I. The molecular β is dependent upon the frequency at which the measurement is performed because of the resonance effect near the absorption peak. A method to compare molecules with different absorption properties by extrapolation of the β value measured at a specific frequency to zero frequency using a two-level model is disclosed by Singer, J. Opt. Soc. Am., B6, 1339-50 (1989). The β value of the extrapolated zero frequency is defined as $\beta_0$. The NLO active molecules of the present invention can exhibit values of the $\beta_0\mu$ product as high as about 1,000 in units of $10^{-48}$ esu measured at a wavelength of 1907 nm.

Thus, it can be appreciated that the present invention provides NLO compounds combining second order nonlinear optical properties with the physical, mechanical and optical properties required of an optical material, together with thermal and chemical stability. The following examples further illustrate the present invention, and are not to be construed as limiting the scope thereof. Unless otherwise indicated, materials were obtained from Aldrich Chemical Supply. All parts and percentages are by weight unless expressly indicated to be otherwise and all temperatures are in degrees Celsius.

EXAMPLES

Example I

Synthesis of 2'-Hydroxy-2-(methyl-sulfinyl)acetophenone

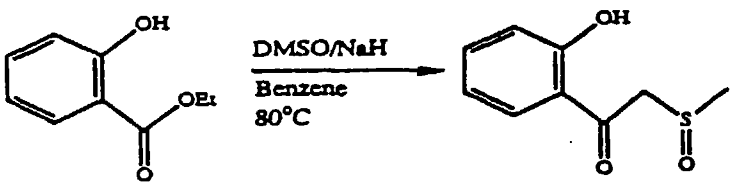


To a solution of dimethylsulfoxide (180 mL, excess) in benzene (400 mL) was added sodium hydride (12.0 gm, 0.5 mol). This suspension was heated to 80°C under an argon atmosphere. After one hour, the resulting solution was cooled to 35°C and a solution of ethyl salicylate (16.6 gm, 0.1 mol) in benzene (200 mL) was added dropwise. After the addition was complete, the mixture was allowed to cool to room temperature over a two-hour period. The benzene was then removed in vacuo and the resulting oil was dissolved in ice water (400 mL). The desired acetophenone was precipitated by dropwise addition of glacial acetic acid while maintaining 0°C. The precipitate was collected by vacuum filtration, and air dried to yield 19.1 gm (96% yield). The pinkish solid was recrystallized from absolute ethanol to yield 15.6 gm (80%).

Example II

Synthesis of 2-[4'-diethylanilino) chromone

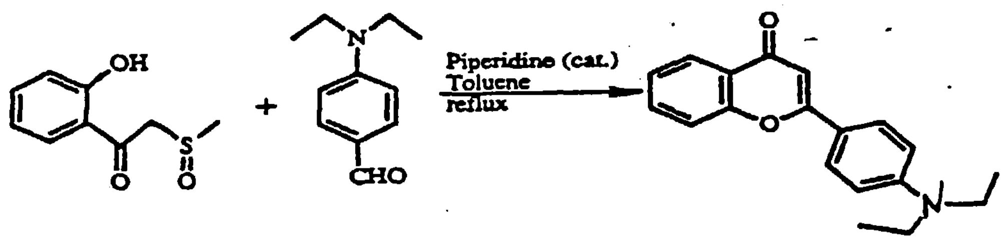


To a solution of the 2'-Hydroxy-2-(methyl-sulfinyl)acetophenone of Example I (1.554 gm, 7.84 mmol) in toluene (50 mL) was added piperidine (5 drops, cat.). The solution was heated to 50°C at which time 4-diethylaminobenzaldehyde (2.78 gm, 15.68 mmol) was added. The resulting mixture was then heated to reflux for three hours. An additional 0.5 gms of 2'-hydroxy-2-(methyl-sulfinyl) acetophenone was then added and the mixture was refluxed for an additional one hour. The solution was cooled, concentrated *in vacuo* and absorbed onto silica (5 gm). This silica mixture was added to a medium pressure column packed with silica. The column was eluded with hexane/ethyl acetate (5:1) with a gradient to 1:1 to afford the chromone (1.26 gm, 86%) as a pale yellow solid and starting aldehyde (1.90 gm).

Example III

Synthesis of 2-(4'-diethylanilino),4-dicyanochromone

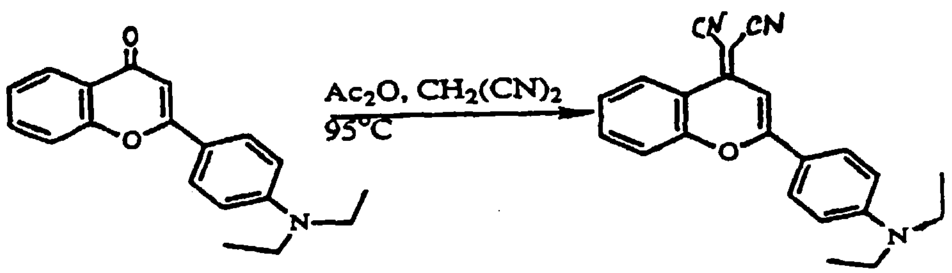


To a 40°C solution of the 2-(4'-diethylanilino) chromone of Example II (1.46 gm, 5.0 mmol) in acetic anhydride (50 mL) was added malononitrile (0.30 gm, 7.7 mmol). The resulting mixture was heated to 95°C and maintained for three hours under an argon atmosphere. The mixture was cooled, concentrated in vacuo to near dryness and the resulting solution was purified by medium pressure flash chromatography using hexane/ethyl acetate (5:1) with a gradient to 3:1 to yield the desired product as a pale yellow solid (2.7 gm, 83%). The compound exhibited a $\lambda_{max}$ (dioxane) of 472 nm and a $\beta\mu$ of 550 ($10^{-48}$esu).

Example IV

Synthesis of 2-(4'-diethylanilino),4-(N,N'-diethylthiobarbituric acid) chromone

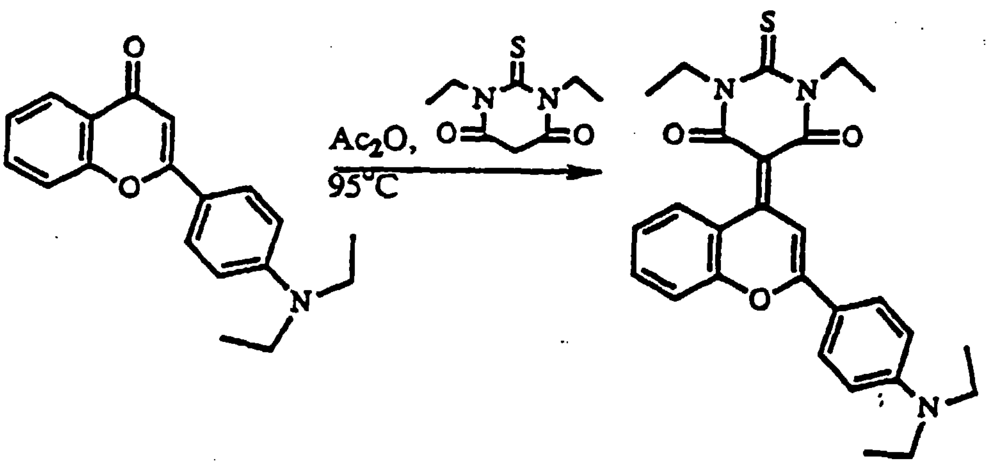


To a 40°C solution of the 2-(4'-diethylanilino) chromone of Example II (1.46 gm, 5.0 mmol) in acetic anhydride (50 mL) was added thiobarbituric acid (0.63 gm, 7.7 mmol). The resulting mixture was heated to 100°C and maintained for three hours under an argon atmosphere. The mixture was cooled, concentrated in vacuo to near dryness and the resulting solution was purified by medium pressure flash chromatography using hexane/ethyl acetate (5:1) with a gradient to 1:1 to yield the desired product as a black solid (2.3 gm, 70%). The compund exhibited a $\lambda_{max}$ (dioxane) of 568 nm and a $\beta\mu$ of 220 ($10^{-48}$esu).

Examples V-XV

The following compounds were prepared as in Examples I-IV with the disclosed yields, $\lambda_{max}$ and $\beta\mu$ ($10^{-48}$ esu) measured at 1907 nm.

| MOLECULE | λmax (dioxane) | βμ | Yield |
|---|---|---|---|
| 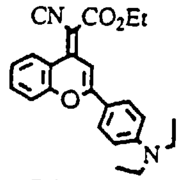 | 450 | 450 | 42% |
| 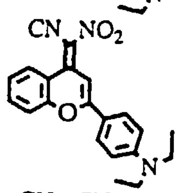 | 488 | 700 | 52% |
| 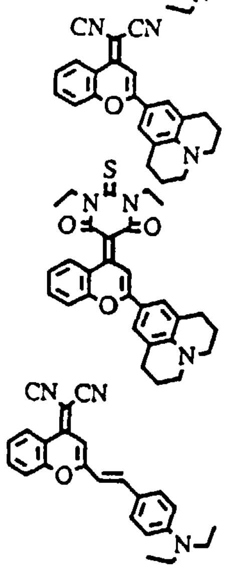 | 490 | 555 | 58% |
| | 589 | 340 | 47% |
| | 503 | 1720 | 79% |

| MOLECULE | λmax (dioxane) | βμ | Yield |
|---|---|---|---|
| 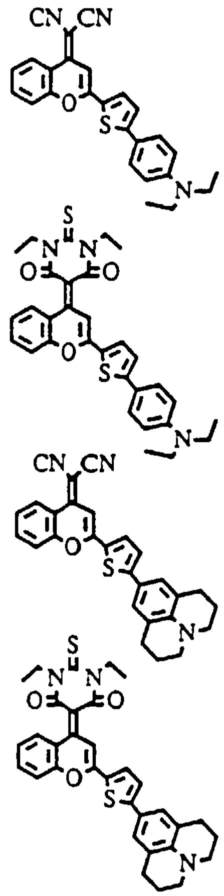 | 505 | 830 | 59% |
| | 615 | 2300 | 84% |
| | 526 | 890 | 57% |
| | 634 | 2350 | 47% |
| 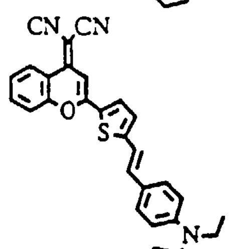 | 602 | 1550 | 32% |
| 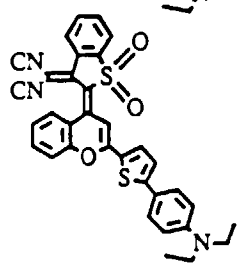 | 628 | 3100 | 56% |

The NLO compounds of the present invention thus possess a combination of NLO properties and thermal and chemical stability heretofore unobtained by the prior art. At the same time, the compounds have good solubility, high laser damage thresholds, are easily synthesized and have well-known and understood chemical properties. The single-ring and fused-ring structures of the present invention containing five-membered heteroaromatic rings represent a versatile family of compounds that can be readily varied to increase their second order NLO properties.

The foregoing examples and description of the preferred embodiment should be taken as illustrating, rather than as limiting, the present invention as defined by the claims. As will be readily appreciated, numerous variations and combinations of the features set forth above can be utilized without departing from the present invention as set forth in the claims. All such modifications are intended to be included within the scope of the following claims.

## Claims

1. A nonlinear optical compound exhibiting second-order nonlinear optical properties and possessing a delocalized resonance configuration having a structure represented by:

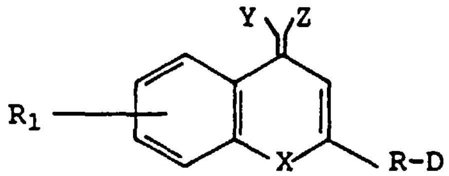

wherein R is a pi-conjugated electron delocalized organic moiety;
D is an electron donating group;
$R_1$ is selected from the group consisting of hydrogen, electron withdrawing moieties, polymer attachment groups, -CN, -COOR", -SOR" and -$SO_2$R", wherein R" is an alkyl group containing from 1 to 15 carbon atoms;
X is S or O; and
Y and Z are independently selected from the group consisting of CN, $CO_2$R', $NO_2$, COR' and $SO_2$R', wherein R' is an alkyl group containing from 1 to 15 carbon atoms, or X and Y together form a ring structure selected from the group consisting of 1,3-indanediones, N,N-diallyl and N,N-dialkyl thiobarbituric acids, 3-dicyanovinylindane-1-ones, 1,3-bisdicyanovinyl indanes and 3-sulfone-1-dicyanovinyl indanes.

2. The nonlinear optical compound of claim 1, wherein R comprises from 1 to 10 aromatic rings or fused ring systems.

3. The nonlinear optical compound of claim 2, wherein R comprises at least one heteroaromatic ring, or at least one fused ring system comprising a heteroaromatic ring.

4. The nonlinear optical compound of claim 3, wherein said heteroaromatic ring comprises a five-membered heteroaromatic ring.

5. The nonlinear optical compound of claim 4, wherein said five-membered heteroaromatic ring has the structure:

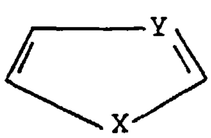

wherein Y is C or N and X is selected from the group consisting of O, S, Se, Te and N.

6. The nonlinear optical compound of claim 5, wherein all of said rings comprise said five-membered heteroaromatic ring, and all of said fused ring systems contain said five-membered heteroaromatic ring.

7. The nonlinear optical compound of claim 2, wherein R comprises two or more rings or fused ring systems linked together to form a delocalized resonance configuration.

8. The nonlinear optical compound of claim 7, wherein R comprises from two to four aromatic rings or fused ring systems.

9. The nonlinear optical compound of claim 7, wherein at least two adjacent rings or fused ring systems are linked together by a conjugated functional group comprising from one to three moieties independently selected from the group consisting of -N=N-, -CH=N-, -CH=N-N=CH- -C≡C and $(-CH=CH-)_j$, wherein j is from one to three.

10. The nonlinear optical compound of claim 7, wherein at least two adjacent rings or fused ring systems are linked together by at least one non-conjugated linkage.

11. The nonlinear optical compound of claim 7, wherein said two or more rings or fused ring systems comprise at least two adjacent rings or fused ring systems directly covalently bonded together without forming a fused structure of said adjacent pair.

12. The nonlinear optical compound of claim 2, wherein said aromatic rings or fused ring systems are independently selected from the group consisting of pyrrole, furan, thiophene, imidazole, oxazole, thiazole, triazole, tetrazole, pyrazole, pyrimidine, purine, quinolines, carbazole, benzene, naphthalene, furazan, pyrazine, indole, isoindole, indazole, phenothiazine, benzotriazole, anthracine, phenanthrene, quinazoline, pteridine and azophenanthrenes.

13. The nonlinear optical compound of claim 12, wherein said aromatic rings or fused rings systems are independently selected from the group consisting of pyrrole, furan, thiophene, thiazole and oxazole.

14. The nonlinear optical compound of claim 2, wherein R comprises at least one fused ring system containing two or three aromatic rings.

15. The nonlinear optical compound of claim 2, wherein R consists essentially of a single fused ring system containing two or three aromatic rings.

16. The nonlinear optical compound of claim 15, wherein said fused ring system comprises at least one five-membered heteroaromatic ring having the structure:

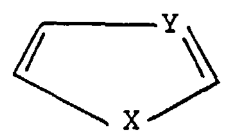

wherein Y is C or N and X is selected from the group consisting of O, S, Se and N.

17. The nonlinear optical compound of claim 16, wherein said fused ring system comprises two or three of said five-membered heteroaromatic rings.

18. The nonlinear optical compound of claim 1, wherein Y and Z are both CN groups.

19. The nonlinear optical compound of claim 1, wherein Y and Z together form a ring structure selected from the group consisting of 1,3-indanediones, N,N-diallyl and N,N-dialkyl thiobarbituric acids, 3-dicyanovinylindane-1-ones, 1,3-bisdicyanovinyl indanes and 3-sulfone-1-dicyanovinyl indanes.

20. The nonlinear optical compound of claim 1, wherein $R_1$ is a polymer attachment group selected from the group consisting of functionalized alkyl moieties containing between 3 and 12 carbon atoms selected from the group consisting of alkoxy, aminoalkyl, alkylhalide, hydroxyalkyl, alkylsulfide, alkylthiol, alkylazide, alkylcarboxylic, alkylsulfonic, alkylalkene, alkylisocyanate, alkylisothiocyanate and alkylalkyne moieties.

21. The nonlinear optical compound of claim 1, wherein D is selected from the group consisting of $-NR_6R_7$, $-OR_8$, $-SR_8$, $-TeR_8$, $-SeR_8$, $-CH=NR_9$, $-CH=N-NR_6R_7$ and $-CH=C[NR_6R_7]_2$, wherein $R_6$ and $R_7$ are independently selected from the group consisting of hydrogen, alkyl groups containing up to 12 carbon atoms and alkyl groups containing up to 12 carbon atoms having reactive functional groups selected from the group consisting of hydroxyl, ethylene, acetylene, amine, thiol, sulfonic acid and carboxylic acid, or $R_6$ and $R_7$ together form a cyclic group containing up to 8 carbon atoms; $R_8$ is selected from the group consisting of alkyl groups containing up 6 carbon atoms; and $R_9$ is selected from the group consisting of hydrogen and alkyl groups containing up to 10 carbon atoms.

22. The nonlinear optical compound of claim 21, wherein $R_6$ and $R_7$ together form a cyclic group selected from the group consisting of pyrrolidine, piperidine, piperazine and morpholine groups.

23. The nonlinear optical compound of claim 1, wherein D is an electron donating group having the structure:

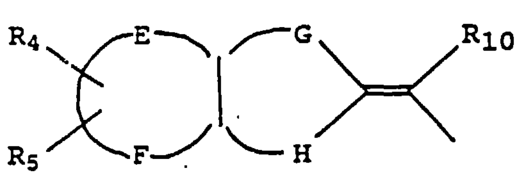

and

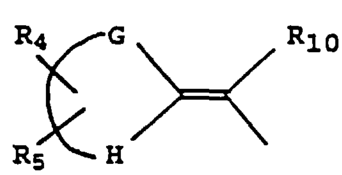

wherein E, F, G and H are members of a saturated or unsaturated five- to eight-membered cyclic ring or two-ring

system having five- to eight-membered rings that are electron donating in nature and are independently selected from the group consisting of -CH-, $-CH_2-$, O, N, S, Se, Te and -NR-; and $R_3$, $R_4$, $R_5$ and $R_{10}$ are independently selected from the group consisting of hydrogen, alkyl moieties containing up to 18 carbon atoms and functionalized alkyl moieties containing up to 18 carbon atoms, wherein said functionalized alkyl moieties are selected from the group consisting of alkoxy, aminoalkyl, alkylhalide, hydroxyalkyl, alkylsulfide, alkylthiol, alkylazide, alkylcarboxylic, alkylsulfonic, alkylalkene, alkylisocyanate, alkylisothiocyanate and alkylalkyne moieties.

**24.** The nonlinear optical compound of claim 23, wherein D comprises an electron donating group selected from the group consisting of 1,3-dithiolium, 2-benzo-1,3-dithiolium and 2-ethylenedithio-1,3-dithiolium moieties.

**25.** The nonlinear optical compound of claim 1, wherein said compound comprises up to two polymer attachment groups independently selected from the group consisting of functionalized alkyl moieties containing between 3 and 12 carbon atoms selected from the group consisting of alkoxy, aminoalkyl, alkylhalide, hydroxyalkyl, alkylsulfide, alkylthiol, alkylazide, alkylcarboxylic, alkylsulfonic, alkylalkyne, alkylisocyanate, alkylisothiocyanate and alkylalkyne moieties.

**26.** The nonlinear optical compound of claim 25, wherein D or $R_1$ is a polymer attachment group, or both D and $R_1$ are polymer attachment groups.

**27.** The nonlinear optical compound of claim 1, further including a second electron donating group attached to R so that said delocalized resonance configuration is maintained.

**28.** A combination exhibiting second order nonlinear optical properties comprising said nonlinear optical compound of claim 1 and a medium chemically inert to said nonlinear optical compound.

**29.** The combination of claim 28, wherein said nonlinear optical compound has an external field-induced molecular alignment.

**30.** The combination of claim 28, comprising a blend of said nonlinear optical compound in a chemically inert medium selected from the group consisting of polyacrylates, polymethacrylates, polyacrylamides, polycarbonates, polyamides, polyquinolines, polyesters, polystyrenes, polyimides, polyether ketones, polyether ether ketones, polyphenylene ethers and copolymers thereof.

**31.** The combination of claim 30, wherein said chemically inert medium comprises a polyimide.

**32.** The combination of claim 28, wherein said nonlinear optical compound comprises at least one polymer attachment group, and said polymer attachment groups are covalently linked to at least one polymer comprising one or more monomeric subunits having a reactive group capable of being covalently attached to a polymer attachment group, whereby said nonlinear optical compound is covalently linked to said polymers at said reactive groups of said subunits via said polymer attachment groups.

**33.** The combination of claim 32, wherein said polymer attachment groups are independently selected from the group consisting of functionalized alkyl moieties containing between 3 and 12 carbon atoms selected from the group consisting of alkoxy, aminoalkyl, alkylhalide, hydroxylalkyl, alkylsulfide, alkylthiol, alkylazide, alkylcarboxylic, alkylsulfonic, alkylalkene, alkylisocyanate, alkylisothiocyanate and alkylalkyne moieties.

**34.** The combination of claim 32, wherein said polymers comprise a plurality of said monomeric subunits having reactive groups covalently substituted with a nonlinear optical compound via said polymer attachment group, so that the ratio of said monomeric subunits having reactive groups covalently linked to a nonlinear optical compound to monomeric subunits without a nonlinear optical compound covalently linked thereto is between about 1:99 and about 50:50.

**35.** The combination of claim 34, wherein said ratio is between about 5:95 and about 40:60.

**36.** The combination of claim 35, wherein said ratio is about 25:75.

**37.** The combination of claim 34, wherein said monomeric subunits having reactive groups covalently substituted with a nonlinear optical compound via a polymer attachment group, and said monomeric subunits without a nonlinear optical compound covalently linked thereto are independently selected from the group consisting of acrylate,

amide, acrylamide, styrene, vinyl halide, acrylonitrile, vinyl alcohol, vinyl acetate monomeric subunits and monomeric subunits of polyimides, polyesters, polyphenylene ethers, polyether ketones, polyether ether ketones, acid anhydrides, polyethylene, polypropylene, polyisobutylene, polyisoprene, polyurethanes, polyquinolines, epoxy polymers, polybenzoxazoles, polybenzothiazoles, polysulfones and polycarbonates.

**38.** The combination of claim 37, wherein said monomeric subunits having reactive groups covalently substituted with a nonlinear optical compound via a functionalize alkyl moiety comprise polyimide monomeric subunits.

**39.** The combination of claim 38, wherein said polyimide is formed by reacting an aromatic diamine with an aromatic dianhydride to form a polyamic acid, which is cyclized to form said polyimide by a thermally induced intramolecular condensation, wherein either of said aromatic diamines or said aromatic dianhydrides, or both, are covalently substituted with said nonlinear optical compound via said functionalized alkyl moiety.

**40.** The combination of claim 28, wherein said chemically inert medium comprises a polymer matrix within which said nonlinear optical compound is cured.

**41.** The combination of claim 40, wherein said polymer matrix comprises a polyimide.

**42.** The combination of claim 40, wherein said polymer matrix is crosslinked.

European Patent Office

# EUROPEAN SEARCH REPORT

Application Number
EP 97 10 0845

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | PATENT ABSTRACTS OF JAPAN vol. 013, no. 342 (P-908), 2 August 1989 & JP 01 102530 A (HITACHI LTD), 20 April 1989, * abstract * | 1 | G02F1/35 |
| Y | DATABASE WPI Section Ch, Week 9202 Derwent Publications Ltd., London, GB; Class E13, AN 92-011638 XP002032638 & JP 03 260 634 A (MITSUBISHI KASEI CORP) , 20 November 1991 * abstract * | 1 | |
| Y | EP 0 602 654 A (ENICHEM SPA) * page 3, line 27 - line 57 * * page 5, line 24 - page 11, line 36 * * page 12, line 43 - page 14, line 9 * | 1-17, 19-23, 25-34, 37,40 | |
| Y | EP 0 637 774 A (ENICHEM SPA) * page 3, line 18 - page 7, line 43 * * page 8, line 46 - page 10, line 5 * | 1-17, 19-34, 37,40 | TECHNICAL FIELDS SEARCHED (Int.Cl.6) G02F |
| Y,P | EP 0 729 056 A (ENICHEM SPA) * page 3, line 33 - page 7, line 22 * | 1-17, 19-34, 37,40 | |
| Y | EP 0 585 999 A (ENICHEM SPA) * page 3, line 52 - page 7, line 16 * | 1-17, 19-34, 37,40 | |

-/--

The present search report has been drawn up for all claims

| Place of search | Date of completion of the search | Examiner |
|---|---|---|
| THE HAGUE | 9 June 1997 | Boulon, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 97 10 0845

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP 0 493 716 A (ENICHEM SPA)<br>* page 3, line 38 - page 8, line 19 *<br>* page 10, line 33 - page 12, line 20 *<br>----- | 1-17, 19-34, 37,40 | |

TECHNICAL FIELDS SEARCHED (Int.Cl.6)

The present search report has been drawn up for all claims

| Place of search | Date of completion of the search | Examiner |
|---|---|---|
| THE HAGUE | 9 June 1997 | Boulon, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)